Europäisches Patentamt

European Patent Office

Office européen des brevets

(11) Publication number: **0 113 520**
A2

## (12) EUROPEAN PATENT APPLICATION

(21) Application number: **83307149.1**

(22) Date of filing: **23.11.83**

(51) Int. Cl.³: **A 61 B 17/34**

(30) Priority: **06.12.82 US 447153**

(43) Date of publication of application:
**18.07.84 Bulletin 84/29**

(84) Designated Contracting States:
**DE FR GB IT**

(71) Applicant: **VANCE PRODUCTS INCORPORATED**
**1100 West Morgan Street**
**Spencer Indiana 47460(US)**

(72) Inventor: **Rutner, Alvin B.**
**1087 Yorkshire**
**Los Altos CA 94922(US)**

(72) Inventor: **Roemer, Frederick D.**
**4790 Woodlands Drive**
**Bloomington, IN 47401(US)**

(74) Representative: **Massey, Alexander et al,**
**MARKS & CLERK Scottish Life House Bridge Street**
**Manchester, M3 3DP(GB)**

(54) **Nephroscopy cannula.**

(57) A nephroscopy cannula apparatus including a cannula having a cylindrical body and defining a central passageway extending between first and second ends of the cannula, a side port portion defining a second passageway extending from the first passageway and to the end of the side port, an apertured disk within which the cannula is received and secured to maintain the cannula in position extending from outside of the body to the interior of the kidney, the disk being sutured to the body for securement purposes, and a septum received over the exposed end of the cannula for at least partially closing that end of the cannula.

Fig.1

EP 0 113 520 A2

1

NEPHROSCOPY CANNULA

## Background of the Invention

Field of the Invention:  The present invention relates to the field of urological equipment and methods, and in particular to a nephroscopy cannula for the provision of a temporary, percutaneous tract into the kidney.

Description of the Prior Art:  Many advances have taken place in the field of urology, and particularly endourology.  There has recently occurred the development of many devices and methods which include percutaneous nephrostomies, the Whitaker test for obstruction, percutaneous placement of ureteral stents, and the percutaneous approaches to the treatment of renal and ureteral calculi, renal abscesses, renal artery stenosis, gonadal varices and renal cancer.  With each advance in endourology, there has been a commensurate decrease in the need for open surgical procedures.  The present invention provides a further advancement directed to the application of techniques which do not require open surgery.

In United States Patent No. 4,177,814, issued to Knepshield et al. on December 11, 1979, there is disclosed a self sealing cannula described for use with surgical instruments such as a trocar or laparoscope.  The

Knepshield et al. device includes an elastomeric valve for providing a seal to the cannula passageway in order to maintain insufflation pressure within the body cavity regardless of the presence or absence of the surgical instrument within the passageway. A urological endoscope, and particularly a resectoscope, is described in United States Patent No. 4,132,227, issued to Ibe on January 2, 1979. The Ibe resectoscope includes a hollow cylindrical sheath for reception of an endoscopic viewing arrangement, and perhaps additionally an operating instrument, as well as clear rinsing water. The resectoscope further includes means for effecting the return flow of the rinsing water discharged from the distal end of the endoscope which improves the field of vision for the operating zone. A urological instrument including a deflecting element is disclosed in United States Patent No. 4,178,920, issued to Cawood et al. on December 18, 1979. The Cawood device includes a deflecting element which is pivotable and is operable with one hand for aid in guiding an instrument such as an endoscope therethrough.

In United States Patent No. 4,237,871, issued to Bonnet on December 9, 1980, there is described a device for the injection of pastes or fluids into the human body, the device being axially displaceable within an endoscope. A safety endoscope with a needle-like trocar and means for signalling completion of a puncture is described in the United States Patent No. 4,254,762, issued to Yoon on March 10, 1981. An intravenous catheter placement device is described in United States Patent No. 4,037,600, issued to Poncy et al. on July 26, 1977. In United States Patent No. 3,878,835, issued to Utsugi on April 22, 1975, there is described a chucking attachment for insertion of a fine, flexible tube into an endoscope, and a dilating apparatus and method are described in the Gravlee et al. patent, issued on May 21, 1974 as United States Patent No. 3,811,449.

3

In contrast to the prior art, the present invention provides a nephroscopy cannula which is temporarily positioned to provide a percutaneous access channel or tract into the renal pelvis of the kidney. In the prior art, such channels or tracts have been provided in conjunction with insertion of a particular device. A device and concept for providing a temporary channel through which various instruments may be repeatedly inserted and retracted has not been proposed.

0113520

4

## Summary of the Invention

Briefly described in one aspect of the present invention there is provided a nephroscopy cannula apparatus including a cannula having a cylindrical portion for extension between the renal pelvis of the kidney and the exterior of the body, the cannula including a central passageway extending the length of the cylindrical portion and also including a second passageway extending to a side port of the cannula, means for temporarily securing the cannula in position relative the body, and means for closing the exposed ends of the cannula.

It is an object of the present invention to provide a temporary, percutaneous access channel into the renal pelvis of the kidney to facilitate repeated insertions and withdrawals of various types of endourological instruments.

It is another object of the present invention to provide an apparatus and method by which repeated access can be obtained to the interior of the kidney, particularly without the need for repeated dilations as have been used in the past.

A further object of the present invention is to provide a nephroscopy cannula having a design which is relatively simple, and yet which adequately performs the functions as previously described.

Further objects and advantages of the present invention will become apparent from the description of the preferred embodiment which follows.

5

## Brief Description of the Drawings

FIG. 1 is a perspective view showing the positioning of the nephroscopy cannula of the present invention with respect to a person's body.

FIG. 2 is a side, elevational view, partially in section, showing the design of the nephroscopy cannula.

FIG. 3 is a top, plan view of a snap-on septum useful with the present invention.

FIG. 4 is a side, cross-sectional view of the septum of FIG. 3.

6

## Description of the Preferred Embodiment

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

Referring in particular to the drawings, there is shown a nephroscopy cannula apparatus 10 constructed in accordance with the present invention. The apparatus 10 is useful for providing a percutaneous access tract to the interior of the kidney, particularly the renal pelvis of the kidney, for repeated insertion and withdrawal of various instruments therethrough. The apparatus includes a cannula 11 having a cylindrical portion 12 and a larger, non-inserted portion 13. A side port portion 14 forms an integral part of the cannula 11 and is positioned in the area of the enlarged portion 13.

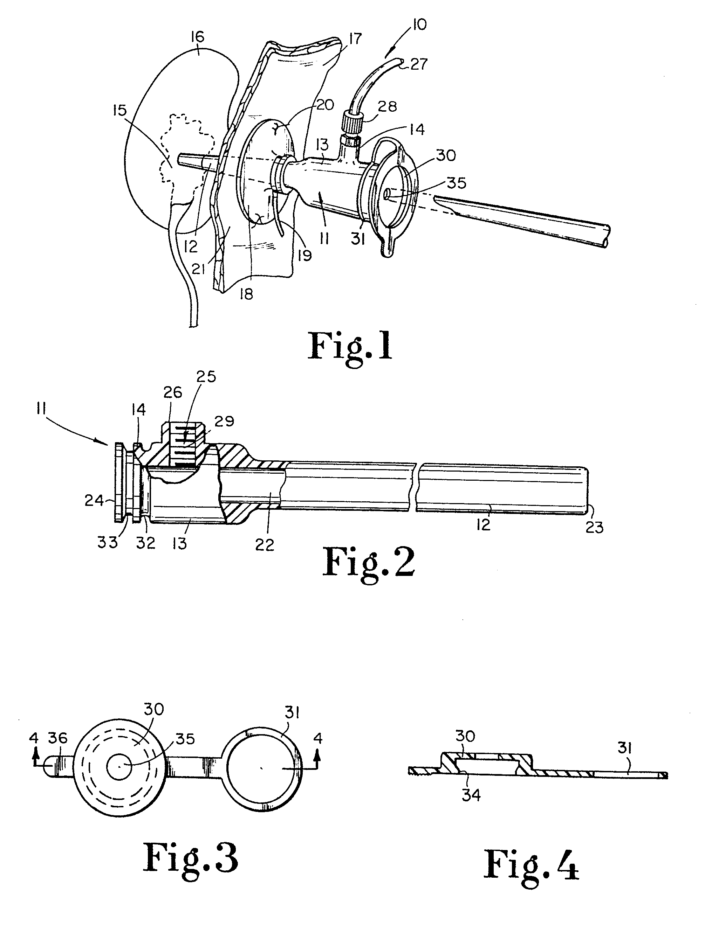

As show particularly in FIG. 1, the cannula includes a first end which is received within the renal pelvis 15 of the kidney 16 and the cylindrical portion 12 extends from that first end to a point exterior of the body. A retention disk 18 is utilized to temporarily secure the cannula in the indicated position. In particular, the disk 18 includes a central opening within which the cylindrical portion 12 is received, and a tie down 19 is used to secure the inner portion of the disk in a firm, sealed manner to the cylindrical portion. In addition, the perimeter of the disk is secured by sutures 20 to the skin 21 of the body adjacent the point of insertion of the cylindrical portion of the cannula. In this fashion, means are provided for temporarily securing the cannula in position with the insertion portion 12 being received with its end within the renal pelvis and with the enlarged portion 13 being received outside of the body.

As shown particularly in FIG. 2, the cannula 11 includes a generally cylindrical body which in particular comprises the inserted portion 12 and enlarged portion 13. This cylindrical body defines a central passageway 22 which extends from the inserted end 23 to the exposed end 24. The side port portion 14 is positioned adjacent the end 24 of the cylindrical body and defines a second passageway 25 which extends from the central passageway 22 to an end 26 of the side port portion. As depicted in FIG. 1, the side port portion 14 is adapted for connection with tubing 27 or the like through a fitting 28 which is received by an internally threaded portion 29. This tubing may be useful, for example, for the passage of liquids therethrough.

The nephroscopy cannula 11 is sized in appropriate dimensions to function in the manner as shown in FIG. 1. The inserted portion 12 has a length and diameter appropriate for reception between the renal pelvis of the kidney and the exterior of the body, and preferably has an outside diameter of from about 7 mm to about 12 mm. The central passageway 22 has a size sufficient for the reception therein of at least one of a number of endourological instruments, such as those instruments selected from the group consisting of cystoscope, nephroscope, endoscope, resectoscope, choledocoscope, panendoscope and nephrocholedocoscope. In particular, the central passageway preferably has a diameter of from about 6 mm to about 10 mm.

As a part of the present invention there is also provided a means for selectively closing at least a portion of the second end 24 of the cannula 11. There is preferably included a septum 30 which is receivable over the end 24 of the cannula. In a preferred embodiment, the septum is integrally formed with a ring 31 and the enlarged portion 13 of the cannula is provided with a pair of circumferential grooves 32 and 33. The ring 31 is received within the groove 32 located farthest from the end 24 of the cannula, as shown for example in FIG. 1. The septum is then provided with a circumferential, inwardly-directed flange 34 (FIG. 4) which is received within the outer groove 33 to provide a snap fit of the septum onto and over the end of the cannula. The septum 30 may be provided as either a solid cover for the end 24, or may have any of a variety of holes, such as the hole 35, for the selective reception of correspondingly sized endourological equipment. For example, the central hole may have a diameter of 0.22, 0.26 or 0.30 inches for the corresponding reception of endourological instruments having outer diameters of 0.236 (18 French), 0.276 (21 French) or 0.315 inches (24 French), respectively. In this fashion, a septum having the appropriately sized central opening 35 may be installed over the end of the cannula 11 to provide for the insertion of an item of equipment, such as an endoscope (FIG. 1), within the central passageway 22 while also providing a seal with respect to that instrument. Alternatively, the blank or solid septum provides for a complete closure of the end 24 of the cannula when the reception of an instrument is not required. A tab 36 may also be provided on the septum 30 in order to facilitate its installation and removal as desired.

In accordance with the present invention, a method is provided for the performance of endourological procedures which includes the steps of inserting a nephroscopy cannula in the position as indicated, temporarily securing the cannula in position, and selectively closing at least a portion of the exposed end of the cannula for closing off the interior of the cannula or for providing a sealed reception of an endourological device therein. Insertion of the nephroscopy cannula is accomplished by providing a sufficient opening for the cannula in accordance with known techniques involving the insertion of a needle through the skin and into the interior of the kidney, followed by the passage of successively larger dilators over the needle. The cannula is then passed over the final dilator and into position as described. For typical sizing of the nephroscopy cannula, a dilator system progressing from the size of 5 French to 40 French could be used.

With the cannula in position and temporarily secured, there is provided a percutaneous access channel or tract into the kidney for the reception therethrough of a variety of endourological devices. Examples of such devices which may be repeatedly inserted and withdrawn through the nephroscopy cannula would include a cystoscope, both adult and pediatric, a flexible nephroscope, an endoscope, a resectoscope, both adult and pediatric, a flexible choledocoscope, a panendoscope and a nephrocholedocoscope. This permits the repeated access by such instruments to the pelvis, calyces and upper ureter. In contrast to the prior art, the present invention allows for the repeated insertion and removal of a variety of devices by which percutaneous access to the kidneys is accomplished. In the past, flexible nephroscopes or nephrocholedocosopes were the devices utilized for percutaneous access to the kidneys, and prior to the present invention the repeated insertion and removal could not be accomplished without accompanying repeated dilations.

It will be appreciated that a variety of materials may be used in the construction of the various elements of the present invention. Such materials are well known in the art, and additional detail is therefore not considered to be necessary.

The diameter of the central passageway is preferably sized for the reception of standard endourological devices. Typical devices as used for adults include endoscopes as exemplified by cystoscopes, panendoscopes, resectoscopes, flexible nephroscopes and choledoconephroscopes, having an outside diameter of from 0.236 inches (18 French) to 0.315 inches (24 French). Similar such devices typically used in pediatric cases would range from an outside diameter of 0.131 inches (10 French) to 0.210 inches (16 French). The diameter of the central passageway 22 is therefore preferably sized to receive such devices, and in the preferred embodiment the central passageway has a diameter of about 0.365 inches (28 French), with the outside diameter of the insertion portion 12 being about 0.450 inches (34.5 French). The overall length of the device is preferably about 6.375 inches (16.2 mm), with the insertion portion spanning a distance of about 5.0625 inches (12.9 mm). The outer diameter of the enlarged portion 13 is preferably about 0.875 inches and the outer diameter of the side port portion 14 is preferably about 0.5 inches.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

11

WHAT IS CLAIMED IS:

1. A nephroscopy cannula apparatus for providing a percutaneous access tract to the interior of the kidney for repeated insertion and withdrawal of various instruments therethrough, said apparatus comprising:

a cannula including a cylindrical portion having a first, central passageway extending from a first end of said cannula to a second end of said cannula, the central passageway of the cylindrical portion having a size sufficient for the reception therein of at least one of the instruments selected from the group consisting of a cystoscope, nephroscope, endoscope, resectoscope, choledocoscope, panendoscope and nephrocholedocoscope, said cannula further including a side port portion adjacent the second end of the cylindrical portion and including a third end separate from the ends of the cylindrical portion, the side port portion defining a second passageway extending from the first passageway to the third end;

the cylindrical portion of said cannula including an insertion portion adjacent the first end which is sized for extending from the renal pelvis of the kidney of the body to the exterior of the body, the cylindrical portion of said cannula further including a non-insertion portion having an outer diameter substantially greater than the outer diameter of the insertion portion of the cylindrical portion;

means for temporarily securing said cannula in position with the insertion portion of the cylindrical portion being received extending from the renal pelvis of the kidney to the exterior of a body; and

means for selectively closing at least a portion of the second end of said cannula.

2. The apparatus of claim 1 in which the central passageway has a diameter of from about 6 mm. to about 10 mm.

3. The apparatus of claim 2 in which the cylindrical portion of said cannula has an outside diameter of from about 7 mm. to about 12 mm.

4. The apparatus of claim 1 in which said means. for closing the second end of the cylindrical portion comprises a septum receivable over the second end of the cylindrical portion.

5. The apparatus of claim 4 in which the cylindrical portion of said cannula includes a pair of circumferential grooves adjacent the second end, said closing means including a circular septum integrally attached to a ring, the ring being received within the circumferential groove farthest from the second end of the cannula, the septum including a circumferential, inwardly-directed flange received within the other of the circumferential grooves and the septum being attached to the cylindrical portion thereby.

6. The apparatus of claim 1 in which said means for securing said cannula comprises a means for securing said cannula to the skin of the body adjacent the point of insertion of the cylindrical portion of said cannula.

7. The apparatus of claim 6 in which said securing means comprises a disc and further includes first attachment means for attaching the disc to said cannula and second attachment means for attaching the disc to the skin of the body adjacent the point of insertion of the cylindrical portion of said cannula.

8. The apparatus of claim 6 in which said means for closing the second end of the cylindrical portion comprises a septum receivable over the second end of the cylindrical portion.

9. The apparatus of claim 8 in which the cylindrical portion of said cannula includes a pair of circumferential grooves adjacent the second end, said closing means including a circular septum integrally attached to a ring, the ring being received within the circumferential groove farthest from the second end of the cannula, the septum including a circumferential, inwardly-directed flange received within the other of the circumferential grooves and the septum being attached to the cylindrical portion thereby.

10. The apparatus of claim 8 in which the side port portion of said cannula includes an internally threaded portion.

11. A nephroscopy cannula apparatus for providing a percutaneous access tract to the interior of the kidney for repeated insertion and withdrawal of various instruments therethrough, said apparatus consisting essentially of:
a cannula including a cylindrical portion having a first, central passageway extending from a first end of said cannula to a second end of said cannula, said cannula further including a side port portion adjacent the second end of the cylindrical portion and including a third end separate from the ends of the cylindrical portion, the side port portion defining a second passageway extending from the first passageway to the third end, the cylindrical portion of said cannula including an insertion portion adjacent the first end which is sized and for extending from the renal pelvis of the kidney of a body to the exterior of the body, the central passageway of the cylindrical portion having a size sufficient for the reception therein of at least one of the instruments selected from the group consisting of a cystoscope, nephroscope, endoscope, resectoscope, choledocoscope, panendoscope and nephrocholedocoscope;

means for temporarily securing said cannula in position with the insertion portion of the cylindrical portion being received extending from the renal pelvis of the kidney to the exterior of a body; and

means for selectively closing at least a portion of the second end of said cannula.

12. The apparatus of claim 11 in which the central passageway has a diameter of from about 6 mm. to about 10 mm.

13. The apparatus of claim 12 in which the 'cylindrical portion of said cannula has an outside diameter of from about 7 mm. to about 12 mm.

14. The apparatus of claim 13 in which the cylindrical portion of said cannula includes a pair of circumferential grooves adjacent the second end, said closing means including a circular septum integrally attached to a ring, the ring being received within the circumferential groove farthest from the second end of the cannula, the septum including a circumferential, inwardly-directed flange received within the other of the circumferential grooves and the septum being attached to the cylindrical portion thereby.

15. The apparatus of claim 14 in which said means for securing said cannula comprises a means for securing said cannula to the skin of the body adjacent the point of insertion of the cylindrical portion of said cannula.

16. A method for the performance of endourological procedures which comprises the steps of:

a. inserting into the body a nephroscopy cannula to have a first end located within the renal pelvis of the kidney and a second end exterior of the body, the cannula including a cylindrical portion having a first, central passageway extending from the first end of said cannula to the second end of said cannula, said cannula further including a side port portion adjacent the second end of the cylindrical portion and including a third end separate from the ends of the cylindrical portion, the side port portion defining a second passageway extending from the first passageway to the third end, the cylindrical portion of said cannula including an insertion portion adjacent the first end which is sized for extending from the renal pelvis of the kidney to the exterior of the body, the central passageway of the cylindrical portion having a size sufficient for the reception therein of at least one of the instruments selected from the group consisting of a cystoscope, nephroscope, endoscope, resectoscope, choledocoscope, panendoscope and nephrocholedocoscope;

b. temporarily securing said cannula in position with the insertion portion of the cylindrical portion being received extending from the renal pelvis of the kidney to the exterior of a body; and

c. selectively closing at least a portion of the second end of said cannula.

17. The method of claim 16 in which said securing of step b. comprises attaching a disc having a central opening to have the central opening secured about and to the cylindrical portion of said cannula and to have the perimeter of the disc attached to the skin of the body adjacent the point of insertion of the cylindrical portion of said cannula.

Fig.1

Fig.2

Fig.3

Fig.4